# NEUE EUROPÄISCHE PATENTSCHRIFT

(11) **EP 3 030 218 B2**
(45) Veröffentlichungstag und Bekanntmachung des Hinweises auf die Entscheidung über den Einspruch: **19.06.2024**
(45) Hinweis auf die Patenterteilung: 30.05.2018
(21) Anmeldenummer: 14730831.6
(22) Anmeldetag: 11.06.2014
(51) Int. Cl.: A61K 8/34, A61K 8/73, A61Q 17/04, A61K 8/04

(54) **GELFÖRMIGES SONNENSCHUTZMITTEL MIT FETTALKOHOLEN**
GEL-LIKE SUN PROTECTION PRODUCT CONTAINING FATTY ALCOHOLS
PRODUIT DE PROTECTION SOLAIRE SOUS FORME DE GEL COMPRENANT DES ALCOOLS GRAS

(30) Priorität: 09.08.2013 DE 102013215828
(43) Veröffentlichungstag der Anmeldung: 15.06.2016
(73) Patentinhaber: Beiersdorf AG, 22529 Hamburg (DE)
(72) Erfinder: BORCHERS, Kathrin, 21614 Buxtehude (DE); TESCH, Mirko, 22303 Hamburg (DE); BLECKMANN, Andreas, 22926 Ahrensburg (DE); MEYER, Christiane, 20357 Hamburg (DE)
(74) Vertreter: Beiersdorf AG
(86) Internationale Anmeldenummer: PCT/EP2014/062080
(87) Internationale Veröffentlichungsnummer: WO 2015/018548

(56) Entgegenhaltungen:
- EP-A1- 0 614 656
- EP-A2- 1 913 932
- WO-A1-2012/149355
- WO-A1-2013/172990
- WO-A2-2007/140442
- DE-A1- 19 923 648
- DE-A1-102006 034 531
- FR-A1- 2 333 493
- US-A- 5 436 241
- US-A- 5 451 394
- US-A1- 2004 228 888
- US-A1- 2005 036 971

## Beschreibung

Die vorliegende Erfindung betrifft eine gelförmige kosmetische Zubereitung enthaltend Ethanol, ein oder mehrere UV-Filter, Hydroxypropylcellulose und ein oder mehrere Fettalkohole gewählt aus der Gruppe der Verbindungen Cetylalkohol, Cetearylalkohol, Myristylalkohol, sowie ein Verfahren und die Verwendung der Fettalkohole gewählt aus der Gruppe der Verbindungen Cetylalkohol, Cetearylalkohol, Myristylalkohol in derartigen Zubereitungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine in jüngerer Zeit populär gewordene Produktform von Sonnenschutzmitteln stellen die transparenten Gele dar. Diese werden in der Regel auf Wasser- oder Alkoholbasis formuliert und mit Zellulosederivaten (z.B. Hydroxyethyl- oder Hydroxypropylderivaten), Acrylaten (z.B. Acrylates/C10-30 Alkylacrylates) oder PVP auf eine entsprechende Viskosität hin verdickt.

Nachteilig am Stande der Technik ist jedoch der Umstand, dass es beim Auftragen und Verreiben der Sonnenschutzgele auf der Haut häufig zur so genannten "Röllchenbildung" kommt. Dabei handelt es sich in der Regel um aufgerollten Abrieb von Hautschuppen, Zubereitungsbestandteilen und Schmutz, der nicht nur unästhetisch ist und die Umwelt verunreinigt, sondern darüber hinaus die kosmetische Wirkung der Zubereitung in nicht vorhersehbarer Weise beeinflusst, da mit den Röllchen Zubereitungsbestandteile aus dem Wirkungsbereich entfernt werden.

Es war daher die Aufgabe der vorliegenden Erfindung, sensorisch attraktive, gelförmige kosmetische Zubereitungen (insbesondere Sonnenschutzmittel) zu entwickeln, bei denen die Neigung zur "Röllchenbildung" reduziert ist.

Überraschend gelöst wird die Aufgabe durch eine gelförmige kosmetische Zubereitung enthaltend
a) Ethanol,
b) ein oder mehrere UV-Filter,
c) Hydroxypropylcellulose mit einem Molekulargewicht von kleiner als 1000 kg/mol, gemessen mittels Größenausschluss-Chromatographie,
d) ein oder mehrere Fettalkohole gewählt aus der Gruppe der Verbindungen Cetylalkohol, Cetearylalkohol, Myristylalkohol, dadurch gekennzeichnet, dass die Gesamtmenge (Summe) an Cetylalkohol, Cetearylalkohol und Myristylalkohol in der Zubereitung von 2,5 bis 7,5 Gewichts-%, bezogen auf das Gesamtgewicht der Zubereitung, beträgt.

Überraschend gelöst wird die Aufgabe ferner durch ein Verfahren zur Reduzierung der Röllchenbildung beim Verreiben einer gelförmigen kosmetischen Zubereitung enthaltend Ethanol, ein oder mehrere UV-Filter und Hydroxypropylcellulose mit einem Molekulargewicht von kleiner als 1000 kg/mol, gemessen mittels Größenausschluss-Chromatographie, auf der Haut, dadurch gekennzeichnet, dass der Zubereitung ein oder mehrere Fettalkohole gewählt aus der Gruppe der Verbindungen Cetylalkohol, Cetearylalkohol, Myristylalkohol zugesetzt wird.

Überraschend gelöst wird die Aufgabe nicht zuletzt durch die Verwendung von ein oder mehreren Fettalkoholen gewählt aus der Gruppe der Verbindungen Cetylalkohol, Cetearylalkohol, Myristylalkohol zur Reduzierung der Röllchenbildung beim Verreiben einer gelförmigen kosmetischen Zubereitung enthaltend Ethanol, ein oder mehrere UV-Filter und Hydroxypropylcellulose mit einem Molekulargewicht von kleiner als 1000 kg/mol, gemessen mittels Größenausschluss-Chromatographie, auf der Haut.

Die Begriffe "erfindungsgemäß", "erfindungsgemäß vorteilhaft" betreffen im Rahmen der vorliegenden Offenbarung dabei sowohl die erfindungsgemäße Zubereitung, das erfindungsgemäße Verfahren und die erfindungsgemäße Verwendung, wenn nicht ausdrücklich etwas anderes erwähnt ist.

Formulierungen wie "erfindungsgemäße Zubereitung" beziehen sich also im Rahmen der vorliegenden Beschreibung immer sowohl auf die beanspruchte Zubereitung als auch auf Zubereitungen, die im beanspruchten Verfahren oder in der beanspruchten Verwendung eingesetzt werden, wenn nicht ausdrücklich etwas anderes erwähnt ist.

Zwar kennt der Stand der Technik die EP2066286 und EP 2288418, doch konnten diese Schriften nicht den Weg zur vorliegenden Erfindung weisen. Darüber hinaus kennt der Fachmann die WO2007/140442, US 5451394, EP1913932, DE 19923648 und US 5436241, die ebenfalls nicht den Weg zur vorliegenden Erfindung weisen konnten.

Es ist erfindungsgemäß vorteilhaft, wenn die erfindungsgemäße Zubereitung Ethanol in einer Menge von 25 bis 75 Gewichts-%, bezogen auf das Gesamtgewicht der Zubereitung, enthält. Erfindungsgemäß bevorzugt ist dabei ein Ethanolgehalt von 45 bis 65 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung Hydroxypropylcellulose in einer Menge von 0,3 bis 3,0 Gewichts-%, bezogen auf das Gesamtgewicht der Zubereitung enthält.

Dabei ist es erfindungsgemäß bevorzugt, wenn die Zubereitung Hydroxypropylcellulose in einer Menge von 10 bis 1,5 Gewichts-%, bezogen auf das Gesamtgewicht der Zubereitung enthält.

Es ist dabei erfindungsgemäß bevorzugt, wenn das Molekulargewicht Mw der Hydroxypropylcellulose zwischen 800 und 900 kg/mol beträgt (gemessen mittels Größenausschlusschromatographie).

Diese lässt sich beispielsweise unter dem Handelsnamen Klucel MF erwerben.

Es ist erfindungsgemäß vorteilhaft, wenn die erfindungsgemäße Zubereitung einen oder mehrere UV-Filter gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethy)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; ,4-Bis-{[4-(2-ethyl-hexyloxy)-2-hydroxy]-phenyl)-6-(4-methoxyphenyl)-1,3,5-triazin; 3-(4-Methylbenzyliden)campher, 3-Benzylldencampher; Ethylhexylsalicylat; Terephthalidendicamphersulfonsäure; 4-(tert.-Butyl)-4'-methoxydiben-zoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoäsäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tribiphenyl-4-yl-1,3,5-triazin; Merocyanine; Titandioxid; Zinkoxid, enthält.

Die erfindungsgemäß bevorzugten UV-Filter sind dabei: 4-Bis-{[4-(2-ethyl-hexyloxy)-2-hydroxy]-phenyl}-6-(4-methoxyphenyl)-1,3,5-triazin; Ethylhexylsalicylat; 4-(tert.-Butyl)-4'-methoxydibenzoylmethan; 2-Ethylhexyl-2-cyano-3,3-dlphenylacrylat; 4-Methoxyzimtsäure(2-ethylhexyl)ester; Homomenthylsalicylat; Dioctylbutylamidotriazon (INCI: Diethylhexyl-Butamidotriazone); 3-(4-(2,2-bis Ethoxycarbonylvinyl)-phenoxy)propenyl)-methoxysiloxan / Dimethylsiloxan - Copolymer

Erfindungsgemäß besonders bevorzugte Ausführungsformen der vorliegenden Erfindung sind dadurch gekennzeichnet, dass die Zubereitung frei ist von 3-(4-Methylbenzyliden)-campher und 2-Hydroxy-4-methoxybenzophenon (Oxybenzon).

Es ist erfindungsgemäß vorteilhaft, wenn die erfindungsgemäße Zubereitung UV-Filter in einer Konzentration von 6 bis 35 Gewichts-%, bezogen auf das Gesamtgewicht der Zusammensetzung enthält, wobei ein Gehalt von 25 bis 35 Gewichts-%, bezogen auf das Gesamtgewicht der Zubereitung, erfindungsgemäß bevorzugt ist.

Erfindungsgemäß vorteilhafte Ausführungsformen der vorliegenden Erfindung sind dadurch gekennzeichnet, dass der Wassergehalt, falls vorhanden, der Zubereitung kleiner oder gleich 3 Gewichts-%, bezogen auf das Gesamtgewicht der Zubereitung, beträgt.

Es ist vorteilhaft im Sinne der erfindungsgemäßen Verfahren und Verwendungen, wenn die Gesamtmenge (Summe) an Cetylalkohol, Cetearylalkohol und Myristylalkohol in der Zubereitung von 0,5 bis 7,5 Gewichts-%, bezogen auf das Gesamtgewicht der Zubereitung, beträgt.

Erfindungsgemäß bevorzugt ist es die Gesamtmenge (Summe) dieser Fettalkohole in einer Konzentration von 2,5 bis 5,0 Gewichts-%, bezogen auf das Gesamtgewicht der Zubereitung einzusetzen.

Bei den Fettalkoholen gewählt aus der Gruppe der Verbindungen Cetylalkohol, Cetearylalkohol, Myristylalkohol ist erfindungsgemäß Cetylalkohol bevorzugt als erfindungsgemäßen Fettalkohol einzusetzten.

Bei Einsatz dieses bevorzugten Fettalkohols ist es erfindungsgemäß vorteilhaft, diese im Gewichtsverhältnis von 4 bis 5 Gewichts-% einzusetzen.

Es Ist erfindungsgemäß vorteilhaft, wenn die erfindungsgemäße Zubereitung Isoprpopylstearat, Dicaprylylether, Isopropylpaimitat und/oder Ethylhexylstearat enthält. Dabei Ist es erfindungsgemäß bevorzugt Isoprpopylstearat einzusetzen.

Es ist dabei vorteilhaft im Sinne der vorliegenden Erfindung, wenn die Gesamtmenge (Summe) an Isoprpopy-Istearat, Dicaprylylether, Isopropylpalmitat und Ethylhexylstearat in der Zubereitung von 0,5 bis 2 Gewichts-%, bezogen auf das Gesamtgewicht der Zubereitung, beträgt.

Vorteilhafte Ausführungsformen der erfindungsgemäßen Zubereitung, der erfindungsgemäßen Verwendung und des erfindungsgemäßen Verfahrens sind dadurch gekennzeichnet, dass die Zubereitung eine Viskosität von 1200 bis 3000 m Pas. Die Messung erfolgte bei 25°C mithilfe eines Kegel-Platte Viskosimeter mit einem Durchmesser von 40mm und mit einer Scherrate von 10s-1.

Es ist erfindungsgemäß besonders vorteilhaft, wenn die erfindungsgemäße Zubereitung transparent ist.

Dabei gilt eine Zubereitung erfindungsgemäß und anspruchsgemäß als transparent, wenn es möglich ist, bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen (Schrifttyp Arial Schriftgröße 10), die sich unmittelbar hinter der Einmal-Küvette befinden, sollten erkennbar und lesbar sein.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

Darüber hinaus kann die erfindungsgemäße Zubereitung vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl-oder-monobutylether, Propylenglykolmonomethyl, -monoethyl- oder -monobutylether, Diethylenglykolmonomethyl- oder -monoethylether und analoge Produkte.

Erfindungsgemäß vorteilhaft enthält die erfindungsgemäße Zubereitung ein oder mehrere Parfümstoffe.

Erfindungsgemäß bevorzugt enthält die erfindungsgemäße Zubereitung ein oder mehreren Parfümstoffen gewählt aus der Gruppe 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin, Limonen [5989-27-5], Citral, Linalool [78-70-6], alpha-Isomethylionon [1335-46-2], Geraniol [106-24-1], Citronellol [1 06-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] und [127-51-5].

In einer erfindungsgemäß vorteilhaften Ausführungsform ist die erfindungsgemäße Zubereitung frei von C12-13 Pareth-9.

Die erfindungsgemäßen Zubereitungen können beispielsweise als Sonnenschutzmittel oder Tagespflegeprodukt verwendet werden.

Die erfindungsgemäßen Zubereitungen können auch als Spray oder Tränkung eingesetzt werden.

### Vergleichsversuche

| **Rohstoff-INCI** | **m [%]** | **m [%]** |
|---|---|---|
| Butyl Methoxydibenzoylmethane | 4,50 | 4,50 |
| Octocrylene | 9,00 | 9,00 |
| Homosalate | 9,00 | 9,00 |
| Ethylhexyl Salicylate | 4,50 | 4,50 |
| Acrylates/Octylacrylamide Copolymer | 2,00 | 2,00 |
| Cetyl Alcohol | 5,00 | 0,00 |
| Hydroxypropylcellulose (M_{w} 850 kg/mol) | 1,30 | 1,30 |
| Glycerin | 1,00 | 1,00 |
| Parfum | 0,70 | 0,70 |
| Menthol | 0,001 | 0,001 |
| Alcohol Denat. | 62,999 | 67,999 |

Die Formulierung mit Cetylalkohol zeigt im Gegensatz zur Zubereitung ohne Cetylalkohol keine Neigung zur Röllchenbildung.

| **Rohstoff-INCI** | **m [%]** | **m [%]** |
|---|---|---|
| Butyl Methoxydibenzoylmethane | 4,50 | 4,50 |
| Octocrylene | 7,50 | 7,50 |
| Homosalate | 9,50 | 9,50 |
| Ethylhexyl Salicylate | 4,75 | 4,75 |
| Bis-Ethylhexyloxyphenol Methoxyphenyl Triazine | 0,60 | 0,60 |
| Polysilicone-15 | 0,30 | 0,30 |
| Acrylates/Octylacrylamide Copolymer | 2,00 | 2,00 |
| Cetyl Alcohol | 4,00 | 4,00 |
| Hydroxypropylcellulose (M_{w} 850 kg/mol) | 1,30 | 1,30 |
| Isopropyl Stearate | 0,00 | 1,00 |
| Glycerin | 1,00 | 1,00 |
| Parfum | 0,80 | 0,80 |
| Menthol | 0,1 | 0,1 |
| Alcohol Denat. | 63,65 | 62,65 |

Beide Formulierungen zeigen keine Röllchenbildung. Die Zubereitung mit Isopropylstearate zeigt im direkten sensorischen Vergleich deutliche Vorteile auf. Die Formulierung mit lospropylstearate ist weniger klebrig und hinterlässt ein leichteres Hautgefühl als die Formulierung ohne Isopropylstearate.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Rohstoff-INCI** | **m [%]** | **m [%]** | **m [%]** |
|---|---|---|---|
| Butyl Methoxydibenzoylmethane | 4,50 | 4,50 | 4,75 |
| Octocrylene | 7,50 | 9,50 | 9,50 |
| Homosalate | 9,50 | 9,50 | 9,50 |
| Ethylhexyl Salicylate | 4,75 | 4,75 | 4,75 |
| Bis-Ethylhexyloxyphenol Methoxyphenyl Triazine | 0,60 | 2,25 | 2,25 |
| Polysilicone-15 | | 1,00 | 1,00 |
| Diethylhexyl Butamido Triazone | | 1,00 | 1,00 |
| Ethylhexyl Methoxycinnamate + BHT | | 0,50 | 0,50 |
| C12-15 Alkyl Benzoate | | 9,50 | 7,50 |
| Acrylates/Octylacrylamide Copolymer | 2,00 | 2,00 | 2,00 |
| Cetyl Alcohol | 4,00 | 5,00 | 5,00 |
| Hydroxypropylcellulose (M_{w} 850 kg/mol) | 1,30 | 1,30 | 1,30 |
| Isopropyl Stearate | 1,00 | | |
| Glycerin | 1,00 | 1,00 | 1,00 |
| Parfum | 0,80 | 0,80 | 0,80 |
| Menthol | 0,01 | 0,01 | 0,01 |
| Alcohol Denat. | 63,04 | 47,39 | 49,14 |

## Patentansprüche

1. Gelförmige kosmetische Zubereitung enthaltend
a) Ethanol,
b) ein oder mehrere UV-Filter,
c) Hydroxypropylcellulose mit einem Molekulargewicht von kleiner als 1000 kg/mol, gemessen mittels Größenausschlusschromatographie,
d) ein oder mehrere Fettalkohole gewählt aus der Gruppe der Verbindungen Cetylalkohol, Cetearylalkohol, Myristylalkohol, **dadurch gekennzeichnet, dass** die Gesamtmenge (Summe) an Cetylalkohol, Cetearylalkohol und Myristylalkohol in der Zubereitung von 2,5 bis 7,5 Gewichts-%, bezogen auf das Gesamtgewicht der Zubereitung, beträgt.

2. Verfahren zur Reduzierung der Röllchenbildung beim Verreiben einer gelförmigen kosmetischen Zubereitung enthaltend Ethanol, ein oder mehrere UV-Filter und Hydroxypropylcellulose, mit einem Molekulargewicht von kleiner als 1000 kg/mol, gemessen mittels Größenausschlusschromatographie, auf der Haut, **dadurch gekennzeichnet, dass** der Zubereitung ein oder mehrere Fettalkohole gewählt aus der Gruppe der Verbindungen Cetylalkohol, Cetearylalkohol, Myristylalkohol zugesetzt wird.

3. Verwendung von ein oder mehreren Fettalkoholen gewählt aus der Gruppe der Verbindungen Cetylalkohol, Cetearylalkohol, Myristylalkohol zur Reduzierung der Röllchenbildung beim Verreiben einer gelförmigen kosmetischen Zubereitung enthaltend Ethanol, ein oder mehrere UV-Filter und Hydroxypropylcellulose, mit einem Molekulargewicht von kleiner als 1000 kg/mol, gemessen mittels Größenausschlusschromatographie, auf der Haut.

4. Zubereitung nach Anspruch 1, Verfahren nach Anspruch 2 oder Verwendung nach Anspruch 3, **dadurch gekennzeichnet, dass** die Zubereitung Ethanol in einer Menge von 25 bis 75 Gewichts-%, bezogen auf das Gesamtgewicht der Zubereitung, enthält.

5. Zubereitung, Verfahren oder Verwendung nach einem der vorhergehenden Ansprüche, **dadurch gekennzeichnet, dass** die Zubereitung Hydroxypropylcellulose in einer Menge von 0,3 bis 3,0 Gewichts-%, bezogen auf das Gesamtgewicht der Zubereitung enthält.

6. Zubereitung, Verfahren oder Verwendung nach einem der vorhergehenden Ansprüche, **dadurch gekennzeichnet, dass** die Zubereitung einen oder mehrere UV-Filter gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol, und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; ,4-Bis-{[4-(2-ethyl-hexyloxy)-2-hydroxy]-phenyl}-6-(4-methoxyphenyl)-1,3,5-triazin; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tribiphenyl-4-yl-1,3,5-triazin; Merocyanine; Titandioxid; Zinkoxid, enthält.

7. Zubereitung, Verwendung oder Verfahren nach einem der vorhergehenden Ansprüche, **dadurch gekennzeichnet, dass** der Wassergehalt der Zubereitung kleiner oder gleich 3,0 Gewichts-%, bezogen auf das Gesamtgewicht der Zubereitung, beträgt.

8. Verwendung oder Verfahren nach einem der vorhergehenden Ansprüche, **dadurch gekennzeichnet, dass** die Gesamtmenge (Summe) an Cetylalkohol, Cetearylalkohol und Myristylalkohol in der Zubereitung von 0,5 bis 7,5 Gewichts-%, bezogen auf das Gesamtgewicht der Zubereitung, beträgt.

9. Zubereitung, Verfahren oder Verwendung nach einem der vorhergehenden Ansprüche, **dadurch gekennzeichnet, dass** die Zubereitung Isoprpopylstearat, Dicaprylylether, Isopropylpalmitat und/oder Ethylhexylstearat enthält.

10. Zubereitung, Verwendung oder Verfahren nach Anspruch 9, **dadurch gekennzeichnet, dass** die Gesamtmenge (Summe) an Isoprpopylstearat, Dicaprylylether, Isopropylpalmitat und Ethylhexylstearat in der Zubereitung von 0,5 bis 2,0 Gewichts-%, bezogen auf das Gesamtgewicht der Zubereitung, beträgt.

11. Zubereitung, Verfahren oder Verwendung nach einem der vorhergehenden Ansprüche, **dadurch gekennzeichnet, dass** die Zubereitung eine Viskosität von 1200 bis 3000 mPas (gemessen bei 25°C mithilfe eines Kegel-Platte Viskosimeter mit einem Durchmesser von 40mm und mit einer Scherrate von 10s-1) aufweist.

12. Zubereitung, Verfahren oder Verwendung nach einem der vorhergehenden Ansprüche, **dadurch gekennzeichnet, dass** die Zubereitung transparent ist.

## Claims

1. Cosmetic preparation in gel form, comprising
a) ethanol,
b) one or more UV filters,
c) hydroxypropyl cellulose having a molecular weight of less than 1000 kg/mol, measured by size exclusion chromatography,
d) one or more fatty alcohols selected from the group consisting of the compounds cetyl alcohol, cetearyl alcohol, myristyl alcohol, **characterized in that** the total amount (sum) of cetyl alcohol, cetearyl alcohol and myristyl alcohol in the preparation is from 2.5 to 7.5 wt%, based on the total weight of the preparation.

2. Process for reducing pilling when rubbing a cosmetic preparation in gel form, comprising ethanol, one or more UV filters and hydroxypropyl cellulose having a molecular weight of less than 1000 kg/mol, measured by size exclusion chromatography, onto the skin, **characterized in that** one or more fatty alcohols selected from the group consisting of the compounds cetyl alcohol, cetearyl alcohol and myristyl alcohol have been added to the preparation.

3. Use of one or more fatty alcohols selected from the group consisting of the compounds cetyl alcohol, cetearyl alcohol, myristyl alcohol for reducing pilling when rubbing a cosmetic preparation in gel form, comprising ethanol, one or more UV filters and hydroxypropyl cellulose, having a molecular weight of less than 1000 kg/mol, measured by size exclusion chromatography, onto the skin.

4. Preparation according to Claim 1, process according to Claim 2 or use according to Claim 3, **characterized in that** the preparation contains ethanol in an amount of 25 to 75 wt%, based on the total weight of the preparation.

5. Preparation, process or use according to any of the preceding claims, **characterized in that** the preparation contains hydroxypropyl cellulose in an amount of 0.3 to 3.0 wt%, based on the total weight of the preparation.

6. Preparation, process or use according to any of the preceding claims, **characterized in that** the preparation contains one or more UV filters selected from the group consisting of the compounds 2-phenylbenzimidazole-5-sulfonic acid and/or salts thereof; phenylene-1,4-bis-(2-benzimidazoyl)-3,3',5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis-(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; ,4-bis-{[4-(2-ethylhexyloxy)-2-hydroxy]phenyl}-6-(4-methoxyphenyl)-1,3,5-triazine; 3-(4-methylbenzylidene)camphor; 3-benzylidenecamphor; ethylhexyl salicylate; terephthalidenedicamphorsulfonic acid; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)-benzoate; di(2-ethylhexyl 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzo-phenone; hexyl 2-(4'-diethylamino-2'-hydroxybenzoyl)benzoate; homomenthyl salicylate; 2-ethylhexyl 2-hydroxybenzoate; dimethicodiethyl benzalmalonate; 3-(4-(2,2-bisethoxycarbonylvinyl)phenoxy)propenyl)methoxysiloxane/dimethylsiloxane copolymer; dioctylbutylamidotriazone (INCI: Diethylhexyl Butamidotriazone); 2,4-bis[5-(1-dimethylpropyl)-benzoxazol-2-yl-(4-phenyl)imino]-6-(2-ethylhexyl)imino-1,3,5-triazine having CAS No. 288254-16-0; tris(2-ethylhexyl) 4,4',4"-(1,3,5-triazine-2,4,6-triyltriimino)trisbenzoate) (also: 2,4,6-tris[anilino-(p-carbo-2'-ethyl-1'-hexyloxy)]-1,3,5-triazine (INCI: Ethylhexyl Triazone)); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tribiphenyl-4-yl-1,3,5-triazine; merocyanines, titanium dioxide and zinc oxide.

7. Preparation, use or process according to any of the preceding claims, **characterized in that** the water content of the preparation is less than or equal to 3.0 wt%, based on the total weight of the preparation.

8. Use or process according to any of the preceding claims, **characterized in that** the total content (sum) of cetyl alcohol, cetearyl alcohol and myristyl alcohol in the preparation is from 0.5 to 7.5 wt%, based on the total weight of the preparation.

9. Preparation, process or use according to any of the preceding claims, **characterized in that** the preparation comprises isopropyl stearate, dicaprylyl ether, isopropyl palmitate and/or ethylhexyl stearate.

10. Preparation, use or process according to Claim 9, **characterized in that** the total content (sum) of isopropyl stearate, dicaprylyl ether, isopropyl palmitate and ethylhexyl stearate in the preparation is from 0.5 to 2.0 wt%, based on the total weight of the preparation.

11. Preparation, process or use according to any of the preceding claims, **characterized in that** the preparation has a viscosity of 1200 to 3000 mPas (measured at 25ºC using a cone-plate viscometer having a diameter of 40 mm and a shear rate of 10 s⁻¹).

12. Preparation, process or use according to any of the preceding claims, **characterized in that** the preparation is transparent.

## Revendications

1. Préparation cosmétique sous forme de gel contenant
a) de l'éthanol,
b) un ou plusieurs filtres UV,
c) de l'hydroxypropylcellulose dotée d'un poids moléculaire inférieur à 1 000 kg/mole, mesuré au moyen d'une chromatographie d'exclusion stérique,
d) un ou plusieurs alcools gras choisis dans le groupe des composés alcool cétylique, alcool cétéarylique, alcool myristylique, **caractérisée en ce que** la quantité totale (somme) d'alcool cétylique, d'alcool cétéarylique et d'alcool myristylique dans la préparation est de 2,5 à 7,5 % en poids, par rapport au poids total de la préparation.

2. Procédé pour la réduction de la formation de petites boules lors de l'étalement par frottement d'une préparation cosmétique sous forme de gel contenant de l'éthanol, un ou plusieurs filtres UV et de l'hydroxypropylcellulose, dotée d'un poids moléculaire inférieur à 1 000 kg/mole, mesuré au moyen d'une chromatographie d'exclusion stérique, sur la peau, **caractérisé en ce que** la préparation est additionnée d'un ou plusieurs alcools gras choisis dans le groupe des composés alcool cétylique, alcool cétéarylique, alcool myristylique.

3. Utilisation d'un ou de plusieurs alcools gras choisis dans le groupe des composés alcool cétylique, alcool cétéarylique, alcool myristylique, pour la réduction de la formation de petites boules lors de l'étalement par frottement d'une préparation cosmétique sous forme de gel contenant de l'éthanol, un ou plusieurs filtres UV et de l'hydroxypropylcellulose, dotée d'un poids moléculaire inférieur à 1 000 kg/mole, mesuré au moyen d'une chromatographie d'exclusion stérique, sur la peau.

4. Préparation selon la revendication 1, procédé selon la revendication 2 ou utilisation selon la revendication 3, caractérisé(e) en ce que la préparation contient de l'éthanol en une quantité de 25 à 75 % en poids, par rapport au poids total de la préparation.

5. Préparation, procédé ou utilisation selon l'une quelconque des revendications précédentes, caractérisé(e) en ce que la préparation contient de l'hydroxypropylcellulose en une quantité de 0,3 à 3,0 % en poids, par rapport au poids total de la préparation.

6. Préparation, procédé ou utilisation selon l'une quelconque des revendications précédentes, caractérisé(e) en ce que la préparation contient un ou plusieurs filtres UV, choisis dans le groupe des composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 4-bis-{[4-(2-éthylhexyloxy)-2-hydroxy]-phényl}-6-(4-méthoxyphényl)-1,3,5-triazine ; 3-(4-méthylbenzylidène)camphre ; 3-benzylidènecamphre ; salicylate d'éthylhexyle ; acide téréphtalidènedicamphresulfonique ; 4-(tert-butyl)-4'-méthoxydibenzoylméthane ; 2-cyano-3,3-diphénylacrylate de 2-éthylhexyle ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; salicylate d'homomenthyle ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1-(diméthylpropyl)benzoxazol-2-yl-(4-phényl)-imino]-6-(2-éthylhexyl)-imino-1,3,5-triazine présentant le n° CAS 288254-16-0 ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone)) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tribiphényl-4-yl-1,3,5-triazine ; mérocyanine ; dioxyde de titane ; oxyde de zinc.

7. Préparation, utilisation ou procédé selon l'une quelconque des revendications précédentes, caractérisé(e) en ce que la teneur en eau de la préparation est inférieure ou égale à 3,0 % en poids, par rapport au poids total de la préparation.

8. Utilisation ou procédé selon l'une quelconque des revendications précédentes, caractérisé(e) en ce que la quantité totale (somme) d'alcool cétylique, d'alcool cétéarylique et d'alcool myristylique dans la préparation est de 0,5 à 7,5 % en poids, par rapport au poids total de la préparation.

9. Préparation, procédé ou utilisation selon l'une quelconque des revendications précédentes, caractérisé(e) en ce que la préparation contient du stéarate d'isopropyle, du dicaprylyléther, du palmitate d'isopropyle et/ou du stéarate d'éthylhexyle.

10. Préparation, utilisation ou procédé selon la revendication 9, caractérisé(e) en ce que la quantité totale (somme) de stéarate d'isopropyle, de dicaprylyléther, de palmitate d'isopropyle et de stéarate d'éthylhexyle dans la préparation est de 0,5 à 2,0 % en poids, par rapport au poids total de la préparation.

11. Préparation, procédé ou utilisation selon l'une quelconque des revendications précédentes, caractérisé(e) en ce que la préparation présente une viscosité de 1 200 à 3 000 mPa.s (mesurée à 25 °C à l'aide d'un viscosimètre à cône-plaque présentant un diamètre de 40 mm et une vitesse de cisaillement de 10 s-1).

12. Préparation, procédé ou utilisation selon l'une quelconque des revendications précédentes, caractérisé(e) en ce que la préparation est transparente.
